Europäisches Patentamt

European Patent Office

Office européen des brevets

(11) Publication number: **0 158 435 B1**

# EUROPEAN PATENT SPECIFICATION

(45) Date of publication of patent specification: **25.09.91**

(51) Int. Cl.⁵: **C12N 9/44**, C12P 19/16,
//(C12N9/44,C12R1:22)

(21) Application number: **85301521.2**

(22) Date of filing: **05.03.85**

(54) Thermostable pullulanase possessing a wide operating pH and process for production thereof.

(30) Priority: **07.03.84 JP 43368/84**

(43) Date of publication of application:
**16.10.85 Bulletin 85/42**

(45) Publication of the grant of the patent:
**25.09.91 Bulletin 91/39**

(84) Designated Contracting States:
**BE DE FR GB NL**

(56) References cited:
**EP-A- 0 063 909
FR-A- 2 012 831
FR-A- 2 273 065
GB-A- 1 466 009**

**BIOCHEMISCHE ZEITSCHRIFT, vol. 334, 1961,
pages 79-95; H.BENDER et al.:
"Untersuchungen an Pullulan. II.
Spezifischer Abbau durch ein bakterielles
Enzym"**

(73) Proprietor: **AGENCY OF INDUSTRIAL SCIENCE
& TECHNOLOGY MINISTRY OF INTERNA-
TIONAL TRADE & INDUSTRY
3-1 Kasumigaseki 1-chome Chiyoda-ku
Tokyo(JP)**

(72) Inventor: **Takasaki, Yoshiyuki
63 Shin-Matsudo 7 chome
Matsudo-shi Chiba-ken(JP)**
Inventor: **Yagisawa, Mitsuo
5-1-7-456 Matsubacho
Kashiwa-shi Chiba-ken(JP)**

(74) Representative: **Evans, David Charles et al
F.J. CLEVELAND & COMPANY 40-43, Chan-
cery Lane
London, WC2A 1JQ(GB)**

## Description

This invention relates to a novel thermostable pullulanase possessing a wide optimum operating pH zone and a process for the production thereof.

Pullulanase was first discovered by Bender et al. as an enzyme capable of hydrolyzing pullulan, a polysaccharide produced by Pullularia pullulan, in Aerobacter aerogenese (Biochem. Biophys. Acta., 36, 309 (1959), Japanese Patent Publications SHO 46(1971)-7559, etc.). The subsequent demonstration of the enzyme's ability to hydrolyze the $\alpha$-1,6-glucoside bonds of amylopectin and, when used in combination with $\beta$-amylase, to produce maltose from starch in a high yield has since been attracting growing interest in this enzyme. It is known that enzymes of the same kind are produced by various microorganisms. The enzymes of this kind are called by various designations such as pullulanase and isoamylase. Collectively, they are referred to as $\alpha$-1,6-glucosidase".

Recently $\alpha$-1,6-glucosidase has been found to be effective in enhancing the yield of sugars as in the production of glucose from starch and in the production of maltooligosaccharides such as maltose, maltotriose, maltotertraose, maltopentaose, and maltohexaose from starch as well.

Unfortunately, most conventionally known $\alpha$-1,6-glucosidases such as pullulanase and isoamylase are generally deficient in thermal stability and possess optimum operating temperatures in the range of 45° to 50° C and a narrow operating pH range. Thus, there have been technical and economic obstacles to their indiscriminate effective use in combinations with all the amylases including those enzymes such as glucoamylase which act on the acid side and excel in thermal stability (optimum operating pH in the neighborhood of 4.5 and optimum operating temperature 60° C) and maltooligosaccharide-producing enzymes of microorganic origins having optimum operating pH values in the neutral through alkaline ranges.

Attempts have been made in EP-A-63909 to provide an effective pullulanase. This is derived from a bacterium of the genus Bacillus and has an optimum operating temperature over the range 0° to 75° C. We have now found the above problems may be alleviated by utilizing a pullulanase produced by Klebsiella pneumoniae to provide an optimum operating temperature in the range of 60° C to 63° C, an operating pH value in the range of 2.5 to 10, and an optimum operating pH in the range of 4.5 to 7.5.

This invention has issued from efforts devoted to overcoming the aforementioned disadvantage of the prior art. It aims to provide a pullulanase having a wide optimum operating pH range and excelling in thermal stability and a process for the production of the enzyme. The thermostable pullulanase provided by this invention offers the advantage that it can be effectively used in combination with all amylases including glucoamylase, $\beta$-amylase, and amylases capable of producing maltotriose and maltooligosaccharides of higher orders and can effectively produce glucose, maltose, etc. from starch.

The procedure for carrying out this invention is described in detail below with reference to drawings which illustrate specific embodiments, in which:-

Figure 1(a) is a graph showing the operating pH data obtained of the thermostable pullulanase of the present invention.

Figure 1(b) is a graph showing the operating temperature data of the pullulanase of the present invention.

Figure 2(a) is a graph showing the thermostability of the pullulanase of the present invention.

Figure 2(b) is a graph showing the pH stability of the pullulanase of the present invention.

Microorganisms widely distributed in the natural world were screened in search of an $\alpha$-1,6-glucosidase having a wide optimum operating pH range and excelling in thermal stability. As a result, the pullulanase produced by a bacterium isolated from soil and identified as Klebsiella pneumoniae has been recognized to be a novel thermostable pullulanase having an optimum operating pH value in an extremely wide range of about 4.5 to 7.5 and an optimum operating temperature on the higher temperature side of about 60° C. This invention has been perfected on the basis of this knowledge. To be specific, this invention relates to a thermostable pullulanase having an optimum operating pH value in a wide range of about 4.5 to about 7.5 and to a method for the production of the thermostable pullulanase, which comprises culturing a strain of Klebsiella pneumoniae in a culture medium and collecting the aforementioned thermostable pullulanase from the fermentatively worked culture medium.

In earlier editions of Bergey's Manual of Determinative Bacteriology, The Williams & Wilkins Co., Klebsiella pneumoniae was classified as Aerobacter aerogenes. In the current 8th edition of the book, Aerobacter aerogenes is embraced in Klebsiella pneumoniae. The fact that bacteria of genus Aerobacter and genus Klebsiella produce pullulanase was known to the art. For example, the pullulanase produced by Aerobacter aerogenes has been mentioned in Biochem. Z., 334, 79 (1961), Method in Enzymology, VIII, 555 (1966), Japanese Patent Publication SHO 46(1971)-7559, Agric. Biol. Chem., 37, 2821 (1973), etc. The $\alpha$-1,6-glucosidase produced by bacteria of genus Klebsiella including Klebsiella pneumoniae has been mentioned in Japanese Patent Publication SHO 51(1976)-5072, Japanese Patent Publication SHO 58(1983)-22197, etc. The enzymes disclosed in these references and patent publications are invariably deficient in thermal stability. In Biochem. Z., 334, 79 (1961) and Method in Enzymology, VIII, 555 (1966), the optimum operating temperature of the pullulanase from Aerobacter aerogenes is described to be 47.5°C. In Agric. Biol. Chem., 37, 2821 (1973), the optimum operating temperature of the same enzyme is described to be 50°C.

In Japanese Patent Publication SHO 51(1976)-5072, there is a statement to the effect that Klebsiella pneumoniae has its optimum operating temperature in the range of 45° to 50°C and it is substantially inactivated after one hour's standing at 50°C in the absence of a substrate.

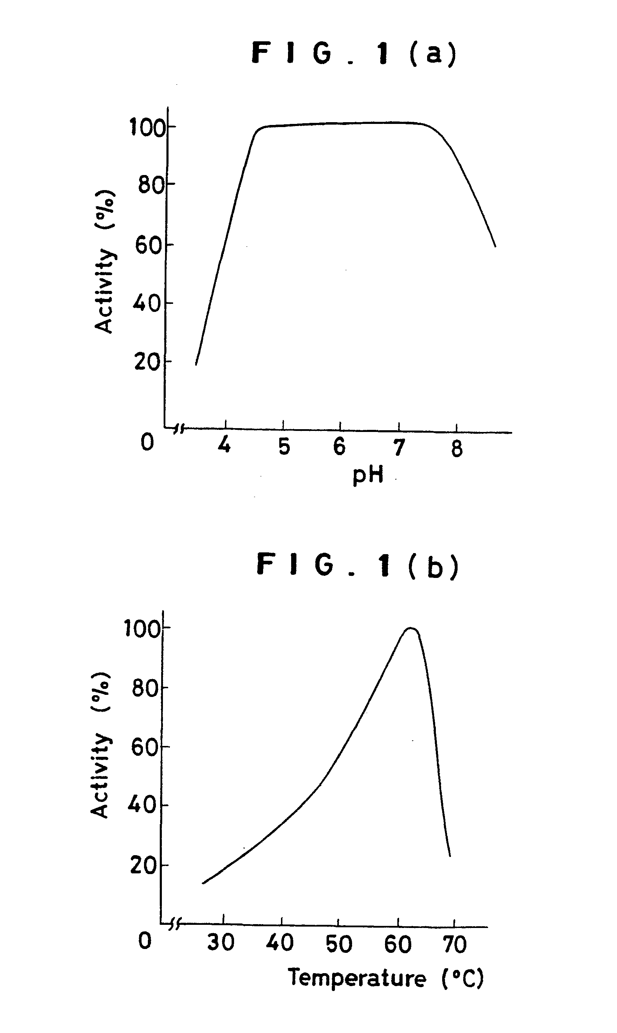

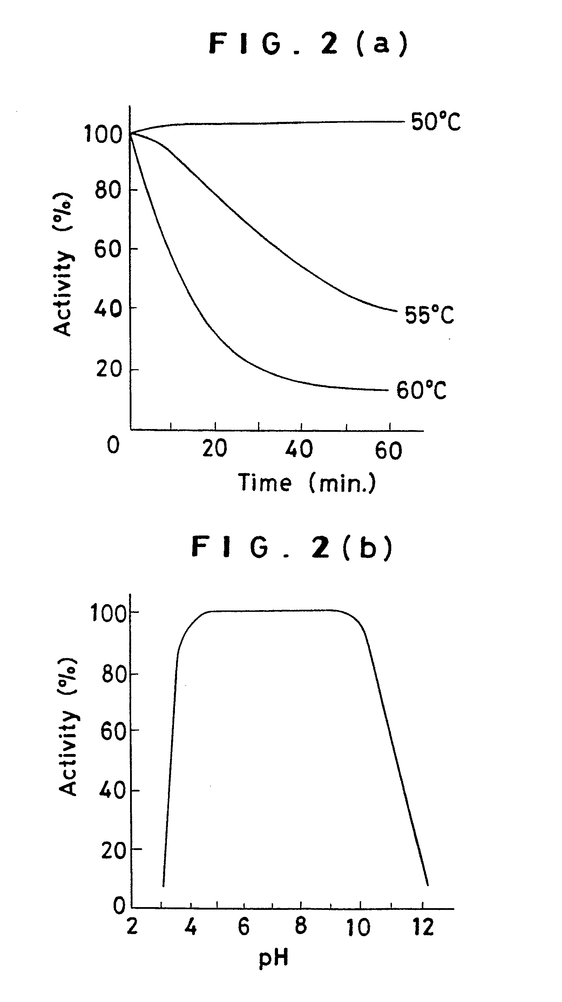

As described above, the heretofore known pullulanases and α-1,6-glucosidases produced by bacteria of genus Aerobacter and genus Klebsiella have been considered to possess optimum operating temperatures in the neighborhood of 45° to 50°C. In contrast, the thermostable pullulanase of the present invention has its optimum operating temperature in the high temperature side range of 60° to 63°C and shows virtually no sign of inactivation even after one hour's heating at 50°C in the absence of a substrate (Figure 1 and Figure 2). Thus, this enzyme excels in thermal stability. Naturally, this enzyme is thermally stabilized in the presence of a substrate. It is also thermally stabilized in the presence of such metal salts as calcium. Even under practical reaction conditions, therefore, the enzyme can undergo a reaction at temperatures of at least 60°C.

As concerns the optimum operating pH, the optimum operating pH of pullulanase and α-1,6-glucosidase from Aerobacter aerogenes and bacteria of genus Klebsiella falls in the neighborhood of 5.0 (Method in Enzymology, VIII, 555 (1966), Japanese Patent Publication SHO 51(1966)-5072, etc.) or in the neighborhood of 6 (Agric. Biol. Chem., 37, 2821 (1973)). In either case, these enzymes are known to suffer heavy loss of activity at a pH value exceeding 6 in particular.

As microorganisms known as capable of producing α-1,6-glucosidase of relatively high thermal stability, there may be cited the pullulanase from genus Bacillus (Japanese Patent Application Public Disclosure SHO 57(1972)-174089 and Starch, 34, 340 (1982)) and the isoamylase from genus Streptomyces (J. Ferment. Technol., 49, 552 (1971)). Although the optimum operating temperatures of the pullulanase and isoamylase produced by these microorganisms fall around 60°C, the enzymes have an optimum operating pH value of 5.0 and suffer serious loss of activity at a pH value above 5.

As described above, the enzyme from genus Aerobacter and genus Klebsiella which were known to the art exhibit poor thermal stability possessing optimum operating temperatures around 50°C and optimum operating pH values in a narrow range around 5.0 or 6.0. In contrast, the thermostable pullulanase of Klebsiella pneumoniae produced by the present invention has an optimum operating pH value in an extremely wide range of about 4.5 to about 7.5 and an optimum operating temperature of 60° to 63°C. Owing to the outstanding thermal stability, it is recognized to be a novel enzyme different from the heretofore known enzymes of genus Aerobacter and genus Klebsiella.

Now, the enzymatic character of the pullulanase of the present invention will be described in detail below.

(1) Action: This enzyme hydrolyzes the α-1,6-glucosidic linkages of pullulan and forms maltotriose. It also hydrolyzes the α-1,6-glucosidic linkages of starch, amylopectin, glycogen, and derivatives thereof.

(2) Operating pH range and optimum operating pH: This enzyme has been found to show its activity in an extremely wide pH range of about 2.5 to about 10 and possess its optimum operating temperature in a wide pH range of about 4.5 to about 7.5 (withstanding 30 minutes' reaction at 50°C in the presence of 2% of pullulan and 0.05M acetate buffer (pH 3 to 5.5), tris buffer (pH 5.5 to 7.5), and tricin buffer (pH 7 to 8.5)).

(3) Operating temperature range and optimum operating temperature: This enzyme has been found to show its activity at temperatures up to about 75°C and possess its optimum operating temperature at around 63°C (withstanding 30 minutes' reaction in the presence of 2% of pullulan and 0.05M acetate buffer (pH 5.0) or 0.05M tris buffer (pH 7.0); Figure 1(b)).

(4) Thermal stability: In an experiment which involved maintaining an aqueous solution of this enzyme at 50°C, 55°C, and 60°C and then testing the solution for residual activity, the enzyme was found to suffer from virtually no loss of activity after one hour's heating at 50°C, loss of about 20% of activity after 20 minutes' heating and loss of about 60% of activity after one hour's heating respectively at 55°C, and loss of about 80% of activity after 30 minutes' heating at 60°C (Figure 2(a)).

(5) pH stability: The enzyme has been found to retain stability at a pH value in the range of about 4 to about 10 (by an experiment involving the steps of allowing the enzyme to stand at room temperature (20°C) for three hours in the presence of 0.1M acetate buffer, phosphate buffer or tris buffer and testing for residual activity; Figure 2 (b)).

(6) Inhibitor: This enzyme undergoes about 93%, about 89%, about 86%, and about 29% of inhibition

respectively by CuSO₄, HgCl₂, ZnSO₄, and FeSO₄ used in a concentration of $1 \times 10^{-1}M$. It undergoes virtually no inhibition by AgNO₃ used in the same concentration.

(7) Procedure for purification: This enzyme can be obtained from the supernatant of the fermentation broth and purified to chromatographic and electrophoretic uniformity by ammonium sulfate fractionation (40 to 70% saturation), DEAE-Sepharose® column chromatography (linear gradient elution at 0 to 0.5M of KCl), and Sephadex G-200® column chromatography.

(8) Molecular weight: The molecular weight of this enzyme as determined by the Sephadex® G-200 gel filtration method is about 120,000.

(9) Procedure of determination of the enzyme activity: This enzyme is added in a suitable amount to 0.5 ml of a 1% pullulan solution (pH 7.0) obtained by dissolving pullulan in 0.1M tris buffer. The mixture is made up to a total volume of 1 ml with water and incubated at 40°C. The amount of this enzyme which is required in producing reducing power equivalent to 1 $\mu$ mole of glucose after one minute standing under the aforementioned conditions is defined as one unit.

The mycological character of the novel thermostable pullulanase-producing bacterium to be used in the present invention will be described below.

(1) Morphological property: A rod-shaped bacterium about 0.8 x about 1.3 $\mu$; generally either independent growth or growth as linked in pairs, no motility, and non-spore-forming.

(2) Characteristics of culture: The bacterium, when cultured on bouillon agar, forms circular colonies of white gloss. The colonies are completely encircled and possess cephalically raised surfaces. The bacterium, when cultured in bouillon medium, grows with uniform turbidity.

(3) Physiological property:

Temperature for growth - The bacterium grows at temperatures up to about 50°C and shows its optimum growth at about 43°C.

pH for growth - The bacterium grows at pH values in the range of about 5 to about 9. It shows its optimum growth in the neighborhood of pH 7.

Gram staining - Negative.

Behavior against oxygen - Facultative anaerobe.

Catalase - Positive.

Oxidase - Negative.

$\beta$-Galactosidase - Positive.

Reduction of nitrates - Positive.

Utility of carbohydrates - The bacterium utilizes glucose, adonitol, L-arabinose, inositol, mannitol, L-rhamnose, etc. and forms acids.

Utility of citric acid - Positive.

Utility of malonic acid - Positive

Methyl red reaction - Negative.

VP reaction - Positive.

Hydrolysis of arginine - Positive.

Liquefaction of gelatin - Negative.

Formation of hydrogen sulfide - Positive.

Indole reaction - Negative.

Decarbonation of lysine - Positive.

Decarbonation of ornithine - Positive.

Urease reaction - Positive.

Formation of phenylpyruvic acid from phenylalanine
- Negative.

From the mycological quality described above, it is judged proper by reference to Bergey's Manual of Determinative Bacteriology, 8th edition (The Williams & Wilkins Co., 1974), to identify this bacterium to be one strain of Klebsiella pneumoniae.

This Klebsiella pneumoniae was deposited at Fermentation Research Institute, Agency of Industrial Science & Technology, Ministry of International Trade & industry, on January 12, 1984, under FERM P-7387, and was also deposited at the same Institute in compliance with the Budapest Treaty on February 26, 1985 and assigned FERM BP-721.

Examples of the nitrogen source utilizable for the production of the novel thermostable pullulanase by this bacterium include organic nitrogen sources such as peptone, meat extract, and corn steep liquor and inorganic nitrogen sources represented by ammonium salts such as urea, ammonium chloride, ammonium sulfate, and ammonium phosphate and nitrates such as sodium nitrate and potassium nitrate. Among the nitrogen sources enumerated above, urea proves a particularly desirable nitrogen source.

As carbon sources, generally corn starch, potato starch, sweet potato starch, glutinous corn starch, glutinous rice starch, and their derived products are usable. Particularly desirable are polysaccharides such as glutinous corn starch, glutinous rice starch, and amylopectin which abound with branched bonds. To a culture medium containing a starchy polysaccharide, lactose is added. The lactose is amply effective when it is added to the medium in an amount of about 0.1%. Generally, when the lactose is added in an amount of about 0.5 to 1%, the amount of pullulanase produced is two to three times as large as when no lactose is added.

Besides the aforementioned nitrogen source and carbon source, phosphate and magnesium salt and small amounts of salts of such metals as calcium and manganese are added as supplements for the culture medium.

The culture is carried out at a pH value in the range of 5 to 9 and a temperature in the range of 20° to 45°C, generally aerobically for one to two days.

The thermostable pullulanase is produced extracellularly and produces substantially no enzyme harmful to the application of the pullulanase such as $\alpha$-amylase. On completion of the culture, therefore, the enzyme can be easily recovered from the culture medium by filtering or centrifuging the culture medium thereby separating cells and recovering the supernatant and concentrating the supernatant, or optionally salting out the enzyme as with ammonium sulfate or sodium sulfate, or precipitating the enzyme by addition of an organic solvent such as acetone, isopropanol, ethanol, or methanol. Commercial production of this enzyme, therefore, can be carried out advantageously from the economic point of view.

Further, since the thermostable pullulanase has its optimum operating pH in a wide range of about 4.5 to about 7.5 and excels in thermal stability as described above, it can be made to react effectively with any of the known amylases, including glucoamylase for producing glucose from starch, $\beta$-amylase of both plant and bacterial origin for producing maltose from starch, amylase from bacteria of genus Bacillus for producing maltotriose (optimum operating pH 6.0 to 6.5), amylase from bacteria of genus Pseudomonas for producing maltotetraose (optimum operating pH 6.5 to 8; Arch. Biochem. Biophys., 145, 105 (1971)), amylase from bacteria of genus Bacillus for producing maltopentaose (optimum operating pH 5 to 8; Arch. Biochem. Biophys., 155, 290 (1973)), and amylase for producing maltohexaose (optimum operating pH 6.8 to 8.0; Biochem. Biophys. Acta., 410, 333 (1975) or Agric. Biol. Chem., 46, 1539 (1982)) under the optimum conditions peculiar to the specific amylase to be used.

The present applicant has proposed a process for producing maltose from starch with enzymes from a bacterium of genus Bacillus producing a composite enzyme combining $\beta$-amylase and $\alpha$-1,6-glucosidase necessary for the production of maltose from starch (British Patent No. 1,466,009). This composite enzyme enables maltose to be produced in a yield of about 88% from various kinds of starch. During the production of maltose by the saccharification of starch by use of the aforementioned composite enzyme, when this saccharification is carried out in the presence of the thermostable pullulanase of this invention, the yield of the maltose increases past 90%. This increase of the yield of maltose is probably explained by the fact that the thermostable pullulanase of the present invention and the $\alpha$-1,6-glucosidase originating in genus Bacillus slightly differ in operating specificity from each other and complement each other in hydrolyzing the $\alpha$-1,6-glucosidic linkages.

The saccharification of starch by the use of the enzyme of this invention is carried out on liquefied starch having a DE (index of the ratio of decomposition of starch, expressed in percentage by the reducing sugar determined as glucose in solids) value of not more than 10 used in a concentration of 10 to 40% at pH 5 to 7 and a temperature in the range of 50° to 60°C.

Now, the present invention will be described more specifically below with reference to working examples.

EXAMPLE 1

In an Erlenmeyer flask having an inner volume of 200 ml, 50 ml of a culture medium containing 0.35% of urea, 0.05% of $K_2HPO_4$, 0.05% of $MgSO_4 \cdot 7H_2O$, 2% of soluble starch, 0.5% of KCl, 5 x $10^{-5}$ M of $MnCl_2$, and 1 x $10^{-3}$ M of $CaCl_2$ was sterilized by the conventional method and, with a strain of Klebsiella pneumoniae FERM BP-721 inoculated thereto, subjected to shaken culture at 30°C for two days. At the end of the culture period, the culture broth was centrifuged to remove cells and obtain the supernatant. The supernatant was tested for the pullulanase activity. Consequently, the activity was found to be 5.6 units per ml of the culture medium.

EXAMPLE 2

The procedure of Example 1 was repeated, except that 2% of amylopectin (from corn starch) was added in place of soluble starch. Consequently, the pullulanase was produced in an amount of 10.8 units per ml of the culture medium.

From the results shown here and those of Example 1, it is noted that the yield of the enzyme is greater when amylopectin is used than when starch is used.

EXAMPLE 3

In a jar fermenter having an inner volume of 5 liters, 3 liters of a culture medium of the same composition as used in Example 1 was sterilized by the conventional method and, with a strain of Klebsiella pneumoniae FERM BP-721 inoculated thereto, subjected to culture under the conditions of 1 liter/min. of aeration rate and 250 rpm of rotational speed. After the culture, the culture broth was centrifuged to remove cells. The thus obtained supernatant was saturated with ammonium sulfate to 70% and the formed precipitate was recovered as crude enzyme. The enzyme obtained was used in combination with amylase in saccharification of starch.

To a liquefied starch of DE 1.4 (containing 1 g as solid), 300 units of a commercially available soybean $\beta$-amylase (as determined by the method proposed in Agric. Biol. Chem., 40, 1515 (1976)) and 1 or 2 units of the pullulanase of Klebsiella pneumoniae obtained by the procedure described above were added and allowed to react thereon at pH 6.0 and 55°C. The sugar composition of the resultant saccharification product was determined by analysis using the high-speed liquid chromatographic method. The results were as shown in Table 1.

## T a b l e   1

| Pullulanase | Glucose | Maltose | Maltotriose | Others |
|---|---|---|---|---|
| None added | 0.0% | 58.4% | 1.2% | 40.4% |
| Pullulanase of this invention added:    1 u/g | 0.0 | 86.0 | 6.3 | 7.7 |
| "    2 u/g | 0.0 | 89.2 | 7.1 | 3.7 |

EXAMPLE 4:

To a liquefied starch of DE 7.7, a commercially available glucoamylase and one unit of the thermostable pullulanase prepared in Example 2 were mixed, adjusted to a solids concentration of 30%, made up to a total volume of 10 ml and left standing at pH 5.0 and 60°C for saccharification of the starch. The sugar composition of the resultant saccharification product was determined by the high-speed liquid gas chromatographic method. The results were as shown in Table 2.

From the table, it is noted that the addition of the pullulanase of this invention enhances the yield of glucose.

In the table, $G_2$ and $G_3$ denote dimer and tremer respectively of glucose.

T a b l e   2

| Pullulanase | Glucose | $G_2$ | $G_3$ or over |
|---|---|---|---|
| None added | 94.6% | 3.3% | 2.1% |
| Pullulanase of this invention | 96.9 | 3.1 | 0.0 |

EXAMPLE 5

A liquefied starch of DE 4.2 (containing 1 g as solid), two units of a maltotriose-producing amylase from Bacillus (sp YT-1004; FERM P-5854), and 1.5 units of the pullulanase of this invention prepared in Example 2 were combined, made up to a total volume of 10 ml with water and left standing at pH 7.0 and 50°C for saccharification of the starch. The sugar composition of the resultant product of saccharification was analyzed. The results were as shown in Table 3.

It is noted from the table that the addition of the pullulanase notably enhances the yield of maltotriose.

T a b l e   3

| Pullulanase | Glucose | Maltose | Maltotriose | Others |
|---|---|---|---|---|
| None added | 2.6% | 14.6% | 58.8% | 24.0% |
| Pullulanase of this invention | 4.0 | 17.8 | 67.7 | 10.5 |

EXAMPLE 6

In an Erlenmeyer flask having an inner volume of 200 ml, 50 ml of a culture medium containing 0.35% of urea, 0.05% of $K_2HPO_4$, 0.05% of $MgSO_4 \cdot 7H_2O$, 2% of soluble starch, 5 x $10^{-5}$M of $MnCl_2$, 2 x $10^{-3}$M of CaCl, and 5 x $10^{-5}$M of $CuSO_4$ and a varying amount, in the range of 0.1 to 1%, of lactose added thereto were sterilized by the conventional method and, with a strain of Klebsiella pnuemoniae inoculated thereto, subjected to shaken culture at 30°C for two days. After the culture period, the cultured broth was centrifuged to remove the cells. The supernatant obtained was tested for the produced pullulanase activity. The results were as shown in Table 4.

T a b l e   4

| Amount of lactose added | Amount of pullulanase produced (in units per ml of medium) |
|:---:|:---:|
| 0 % | 2.50 |
| 0.1 | 2.91 |
| 0.2 | 3.49 |
| 0.5 | 5.59 |
| 0.8 | 7.57 |
| 1.0 | 6.49 |

EXAMPLE 7

In two Erlenmeyer flasks having an inner volume of 200 ml, 50 ml of a culture medium having the same composition as that of the culture medium of Example 6, except that 2% of glucinous rice starch was added in place of soluble starch and 50 ml of a culture medium having the same composition as the first culture medium mentioned above and incorporating additionally therein 0.5% of lactose were separately sterilized by the conventional method and, with a strain of Klebsiella pneumoniae FERM BP-721 inoculated thereto, left standing at 30°C for two days for culture of the strain. After the culture period, the cultured broths were centrifuged to remove the cells and produce supernatants. The supernatants were tested for pullulanase activity. The activity was 6.46 units/ml of culture medium in the sample containing no lactose and 10.8 units/ml of culture medium in the sample containing lactose.

It is noted from the results of Example 6 and Example 7 that addition of lactose to the culture medium containing a starch notably enhances the yield of pullulanase.

EXAMPLE 8

A liquefied starch of DE 1.4 (containing 1 g as solid), 300 units and 30 units (as determined by the method proposed in Agric. Biol. Chem., 48, 1515 (1976)) respectively of $\beta$-amylase and pullulanase produced by Bacillus cereus var. mycoides (FERM P-2391 & ATCC 31102), and 0.5 or 1 unit of the pullulanase of this invention prepared in Example 3 were combined and left standing at pH 6 to 6.5 at a temperature of 50°C for 44 hours for saccharification of the starch. The sugar composition of the resultant product of saccharification was analyzed. The results were as shown in Table 5 below.

It will be noted from the table that addition of one unit of the pullulanase of this invention in combination with the pullulanase from genus Bacillus increases the yield of maltose by 2.9% and decreases the yield of other saccharides.

## T a b l e  5

| Pullulanase of this invention | Glucose | Maltose | Maltotriose | Others |
|---|---|---|---|---|
| None added (units) | 0.0(%) | 88.9(%) | 6.2(%) | 4.9(%) |
| 0.5 | 0.0 | 90.7 | 7.2 | 2.1 |
| 1 | 0.0 | 91.8 | 6.8 | 1.4 |

EXAMPLE 9

A liquefied starch of DE 1.4 (containing 1 g as solid), 300 units and 30 units (determined by the method proposed in the aforementioned literature) respectively of β-amylase and α-1,6-glucosidase (produced by Bacillus cereus var. mycoides, FERM P-2391 in accordance with the procedure of Agric. Biol. Chem., 48, 1515 (1976); the enzymes available from Hokkaido Sugar Co., Ltd. of Japan), and 0.5 unit or 1 unit of the pullulanase of this invention prepared in Example 1 were combined and left standing at pH 6 to 6.5 and 50°C for 44 hours for saccharification of the starch. The sugar composition of the resultant product of saccharification was analyzed. The results were as shown in Table 6.

It is noted from the table that addition of the pullulanase of this invention in combination with the β-amylase and the α-1,6-glucosidase of genus Bacillus lowers the yield of other saccharides and increases the yield of maltose past 90% and that addition of one unit of the pullulanase of this invention increases the yield of maltose by about 3% over the level obtained in the absence of the pullulanase.

Table 6

| β-Amylase | | α-1,6-Glucosidase | | Glucose | Maltose | Maltotriose | Others |
|---|---|---|---|---|---|---|---|
| Genus Bacillus (units) | Soybean (units) | Genus Bacillus (units) | Genus Klebsiella (units) | (%) | (%) | (%) | (%) |
| 300 | 0 | 30 | 0 | 0.0 | 88.9 | 6.7 | 4.9 |
| 300 | 0 | 30 | 0.5 | 0.0 | 90.7 | 7.2 | 2.1 |
| 300 | 0 | 30 | 1.0 | 0.0 | 91.8 | 6.8 | 1.4 |
| 0 | 300 | 0 | 1.0 | 0.0 | 83.2 | 5.8 | 11.0 |
| 0 | 300 | 0 | 2.0 | 0.0 | 88.3 | 7.0 | 4.7 |

## Claims

1. A thermostable pullulanase having an operating temperature in the range of 0° to 75°C characterised in that the pullulanase is obtainable from a strain of Klebsiella pneumoniae and has an optimum operating temperature in the range of 60°C to 63°C, an operating pH value in the range of 2.5 to 10, and an optimum operating pH in the range of 4.5 to 7.5.

2. A process for the production of the thermostable pullulanase of claim 1 characterised by culturing Klebsiella pneumoniae in a culture medium and collecting the pullulanase produced in the culture medium.

3. A process according to claim 2, wherein said Klebsiella pneumoniae is Klebsiella pneumoniae FERM BP-721.

4. A process according to claim 2 or claim 3, wherein said culture is carried out aerobically at pH 5 to 9 and a temperature in the range of 20° to 45° C.

5. A process according to claim 2, claim 3, or claim 4, wherein the culture medium contains a polysaccharide of starch origin.

6. A process according to claim 5, wherein said culture medium further contains lactose.

## Revendications

1. Pullulanase thermostable ayant une température d'efficacité située dans un intervalle allant de 0 à 75° C, caractérisée en ce que la pullulanase peut être obtenue à partir d'une souche de Klebsiella pneumoniae et a une température d'efficacité optimale située dans un intervalle allant de 60° C à 63° C, un pH d'efficacité situé dans un intervalle allant de 2,5 à 10, et un pH d'efficacité optimale dans un intervalle de 4,5 à 7,5.

2. Procédé de production de la pullulanase thermostable selon la revendication 1, caractérisé en ce qu'on cultive la Klebsiella pneumonia dans un milieu de culture et en ce qu'on recueille la pullulanase produite dans le milieu de culture.

3. Procédé selon la revendication 2, dans lequel ladite Klebsiella pneumoniae est la Klebsiella pneumoniae FERM BP-721.

4. Procédé selon la revendication 2 ou la revendication 3, dans lequel ladite culture est effectuée de façon aérobie à un pH de 5 à 9 et à une température située dans un intervalle allant de 20° à 45° C.

5. Procédé selon la revendication 2, la revendication 3 ou la revendication 4, dans lequel le milieu de culture contient un polysaccharide provenant de l'amidon.

6. Procédé selon la revendication 5, dans lequel ledit milieu de culture contient en outre du lactose.

## Patentansprüche

1. Thermostabile Pullulanase mit einer Arbeitstemperatur im Bereich von 0° C bis 75° C, dadurch **gekennzeichnet**, daß die Pullulanase aus einem Klebsiella-pneumoniae-Stamm gewinnbar ist und eine optimale Arbeitstemperatur im Bereich von 60° C bis 63° C, einen Arbeits-pH-Wert im Bereich von 2,5 bis 10 und einen optimalen pH-Wert im Bereich von 4,5 bis 7,5 hat.

2. Verfahren zur Herstellung der thermostabilen Pullulanase gemäß Anspruch 1, dadurch **gekennzeichnet**, daß die Klebsiella pneumoniae in einem Kulturmedium kultiviert und die in diesem Kulturmedium erzeugte Pullulanase gewonnen wird.

3. Verfahren nach Anspruch 2, dadurch **gekennzeichnet**, daß die Klebsiella pneumoniae durch Klebsiella pneumoniae FERM BP-721 dargestellt ist.

4. Verfahren nach Anspruch 2 oder Anspruch 3, dadurch **gekennzeichnet**, daß die Kultur aerobisch bei einem pH-Wert von 5 bis 9 und einer Temperatur im Bereich von 20° C bis 45° C entwickelt wird.

5. Verfahren nach Anspruch 2, 3 oder 4, dadurch **gekennzeichnet**, daß das Kulturmedium ein aus Stärke abgeleitetes Polysaccharid enthält.

6. Verfahren nach Anspruch 5, dadurch **gekennzeichnet**, daß das Kulturmedium außerdem Laktose enthält.

# F I G . 1 ( a )

# F I G . 1 ( b )

# F I G . 2 ( a )

# F I G . 2 ( b )